Europäisches Patentamt

European Patent Office

Office européen des brevets

(11) Numéro de publication: **0 407 276 A2**

## (12) DEMANDE DE BREVET EUROPEEN

(21) Numéro de dépôt: 90401881.9

(22) Date de dépôt: 29.06.90

(51) Int. Cl.⁵: **A61M 11/00**, A61M 11/02, B05B 7/00

(30) Priorité: 04.07.89 FR 8908971

(43) Date de publication de la demande:
09.01.91 Bulletin 91/02

(84) Etats contractants désignés:
AT BE CH DE DK ES FR GB GR IT LI LU NL SE

(71) Demandeur: VALOIS Société Anonyme dite:
Boîte Postale G Le Prieuré
F-27110 Le Neubourg(FR)

(72) Inventeur: Brunet, Michel
La Marnière Carrée
F-27840 Sainte Colombe la
Commanderie(FR)
Inventeur: Jouillat, Claude
La Marette
F-28270 Montigny sur Avre(FR)

(74) Mandataire: Pinguet, André
Cabinet de Propriété Industrielle CAPRI 28
bis, avenue Mozart
F-75016 Paris(FR)

(54) Dispositif de protection et de pulvérisation d'une dose d'un produit divisable.

(57) Dispositif pour projeter et pulvériser une dose d'un produit divisé, en liquide ou en poudre, notamment à usage pharmaceutique, caractérisé en ce qu'il comporte un récipient (1) avec un orifice de sortie (3), une cloison déchirable (2) séparant le récipient en deux chambres, une première chambre (1A) du côté de l'orifice de sortie prévue pour recevoir le produit divisé, et une seconde chambre (1B) comportant au moment de l'emploi un gaz comprimé, un moyen étant prévu pour perforer la cloison au moment de l'emploi.

FIG .1

EP 0 407 276 A2

La présente invention a pour objet un dispositif de projection et de pulvérisation d'une dose d'un produit divisable, en liquide ou en poudre, à des fins médicales. Il est courant d'avoir à introduire dans la gorge ou dans les narines un médicament en poudre ou en liquide. L'opération est délicate, surtout dans les narines, parce qu'il faut renverser la tête en arrière. Malgré les précautions que l'on peut prendre, une partie du médicament peut tomber en dehors de la cible. L'effet attendu ne peut être complètement obtenu, le dosage est aléatoire. La présente invention a pour but un dispositif permettant de chasser le médicament sous forme divisée. Il peut donc être envoyé dans la gorge ou les narines sans pertes, avec une efficacité maximum.

Conformément à la présente invention, ce résultat est obtenu par un dispositif pour projeter et pulvériser une dose d'un produit divisable, en liquide ou en poudre, notamment à usage pharmaceutique, caractérisé en ce qu'il comporte un récipient avec un orifice de sortie, une cloison déchirable séparant le récipient en deux chambres, une première chambre du côté de l'orifice de sortie prévue pour recevoir le produit divisable, et une seconde chambre comportant au moment de l'emploi un gaz comprimé, un moyen étant prévu pour perforer la cloison au moment de l'emploi.

Le moyen pour perforer la cloison peut être une pointe de préférence rainurée, solidaire d'une paroi déformable. En appuyant sur la paroi déformable, on déplace la pointe qui vient perforer la cloison. La déformation de la paroi peut accroître ou créer la pression de gaz dans le second volume en réduisant celui-ci, le gaz étant ainsi comprimé au moment de l'emploi du dispositif. La déformation de la paroi peut être obtenue par la souplesse de la paroi, ou bien la paroi peut comporter une partie cylindrique dans laquelle coulisse un piston. Il est avantageux de faire monter la pression avant de perforer la cloison.

Avantageusement, le récipient peut être réalisé d'une seule pièce. Le récipient peut être en verre ou en plastique.

Avantageusement, la deuxième chambre peut être remplie d'azote à une pression au moins égale à la pression atmosphérique. La première chambre peut, elle aussi, être remplie d'azote en plus du produit à disperser.

Dans une forme particulière de réalisation de l'invention, le récipient se compose:
d'un piston comportant un trou axial le traversant, une rainure périphérique et une lèvre d'étanchéité périphérique;
d'un embout comportant l'orifice de sortie et un moyen élastique pouvant s'encliqueter dans la rainure du piston, l'embout et le piston définissant un logement pouvant recevoir un flacon comportant une paroi latérale et la cloison déchirable, ledit flacon représentant l'enceinte de ladite première chambre contenant le produit à pulvériser, et une surface de contact entre le piston et le flacon étant étanche;

et est caractérisé en ce que la paroi déformable est un cylindre creux doté d'un fond et d'une paroi latérale cylindrique à l'intérieur desquelles coulisse le piston de façon à ce que la lèvre d'étanchéité soit en contact avec ladite paroi latérale sur toute une périphérie, le moyen de perforation étant disposé sur le fond du cylindre de façon à pouvoir passer au travers du trou du piston pour percer la cloison déchirable.

Avantageusement, un ressort peut solliciter le poussoir de façon à l'éloigner du piston.

Un bourrelet périphérique peut éventuellement être formé sur une surface intérieure du poussoir de façon à ce qu'au montage du dispositif, le piston puisse être inséré à l'intérieur du poussoir en forçant la lèvre d'étanchéité à passer sur le bourrelet, et en ce que la sollicitation du ressort additionnée de la force de traction nécessaire à défaire l'encliquetage du moyen élastique de l'embout ne suffise à refaire passer la lèvre d'étanchéité par dessus le bourrelet.

Dans une autre forme particulière de réalisation de l'invention une première extrémité d'un flacon délimitant la première chambre est emboîtée de façon étanche avec frottement à une extrémité de la deuxième chambre, ladite première extrémité du flacon étant fermée par la cloison déchirable, et un embout comportant l'orifice de sortie est emboîté avec frottement sur une deuxième extrémité du flacon. Dans une telle forme de réalisation, dans laquelle la paroi déformable est un piston contre lequel vient appuyer une extrémité d'une tige du poussoir, la seconde chambre étant un cylindre adapté à ce piston, un ressort peut solliciter le piston de façon à l'éloigner du flacon.

Le flacon des deux formes particulières de réalisation décrites ci-dessus peut être en verre ou en plastique.

La cloison déchirable peut être un film thermoscellé. Elle peut être réalisée dans un matériau conducteur de la chaleur. Elle peut occuper toute une section de la première chambre, de façon à présenter une transmissivité thermique maximale.

Dans une forme particulière de l'invention, la première chambre comporte une coupelle cylindrique contenant une poudre à pulvériser et constituée d'un fond et d'une paroi latérale périphérique s'étendant verticalement à partir du fond, ladite paroi latérale périphérique comportant en partie supérieure un moyen d'étanchéité emboîté en force à l'intérieur de la première chambre, ladite paroi latérale périphérique comportant aussi au moins un orifice non radial la traversant et dirigé en biais vers l'intérieur et vers le fond de la coupelle, ledit fond de la coupelle comportant une surface supérieure conique convexe, la coupelle coopérant avec la première chambre de façon à permettre le passage de gaz comprimé de l'intérieur de la première chambre vers l'intérieur de la coupelle par l'intermédiaire du ou des orifice(s) sans que la coupelle ne soit éjectée vers l'extérieur, et le ou les orifice-(s) coopérant avec ladite surface conique pour créer un mouvement tourbillonnaire en même temps que l'expulsion de la substance à pulvériser, lors du passage du gaz comprimé.

Avantageusement, la première chambre contenant le produit à disperser présente une section constante, et l'orifice de sortie est de même section que ladite première chambre.

Le dispositif peut être muni d'un bouchon comportant un couvercle à partir duquel s'étend une jupe jusqu'à une extrémité inférieure, ladite jupe s'emboîtant de façon étanche avec frottement dans la chambre, et est caractérisé en ce que la jupe comporte une fente sur une partie de sa hauteur, à partir de son extrémité inférieure. Une capsule métallique déchirable peut être sertie par dessus le bouchon.

D'autres caractéristiques et avantages de l'invention apparaîtront au cours de la description suivante, donnée à titre d'exemple non limitatif en regard des dessins ci-joints, et qui fera bien comprendre comment l'invention peut être réalisée.

Sur les dessins,
- la figure 1 est une vue en coupe d'un exemple de réalisation de l'invention destiné à la projection d'une poudre,
- la figure 2 est une vue analogue d'un exemple de réalisation destiné à la projection d'un liquide,
- la figure 3 est une vue analogue à la figure 1 pour une variante,
- la figure 4 est une vue en coupe d'une forme particulière de réalisation de l'invention,
- la figure 4a est une vue en coupe du piston du dispositif représenté à la figure 4,
- la figure 5 est une vue en coupe du flacon du dispositif représenté à la figure 4, conditionné pour être stocké,
- la figure 6 est une vue en coupe d'une forme de réalisation de l'invention analogue à celle de la figure 4, le piston et le poussoir n'étant pas représentés,
- la figure 7 est une vue en coupe du flacon du dispositif de la figure 6, conditionné pour être stocké, et
- la figure 8 est une vue en coupe d'une autre forme particulière de réalisation de l'invention.

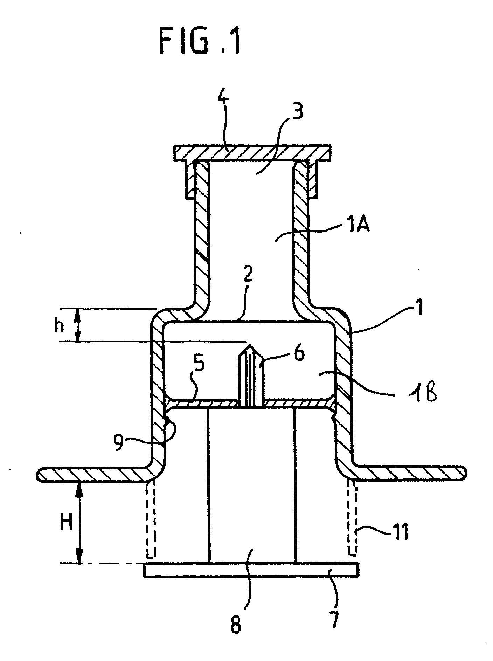

L'exemple de réalisation représenté sur la figure 1 est un petit appareil qui comporte un corps 1, séparé en deux chambres 1A et 1B par une cloison déchirable 2. La première chambre 1A, de petit volume, est placée du côté de l'orifice de sortie 3 qui, avant usage, est normalement fermé par un bouchon 4. Cette chambre est prévue pour recevoir une poudre qui peut occuper tout ou partie du volume de la chambre. La seconde chambre 1B est cylindrique et contient un piston 5 muni d'une pointe rainurée 6, disposée de façon à venir percer la cloison 2 lors du déplacement du piston vers la première chambre. Le piston est commandé par un poussoir 7, avec une tige d'actionnement 8. Le piston peut être maintenu à l'intérieur du cylindre par un bourrelet périphérique 9. Le corps 1 peut être moulé avec une bande déchirable 11, garantissant l'inviolabilité. La seconde chambre 1B peut être chargée initialement avec une certaine surpression de gaz de façon à accroître l'effet de projection.

Avantageusement, la chambre 1B peut être remplie d'azote, à une pression au moins égale à la pression atmosphérique, de façon à garantir la pureté et l'asepsie du gaz contenu dans ladite chambre 1B. La chambre 1A peut, elle aussi, être remplie d'azote en plus du produit à disperser. La bande déchirable 11 peut être moulée avec le poussoir 7, ce qui permet de réaliser le corps 1 en verre aussi bien qu'en plastique.

Cet appareil fonctionne de la façon suivante.

Après arrachement de la bande 11, l'utilisateur enlève le bouchon 4 et place l'appareil en position pour l'application recherchée (par exemple engagement de l'extrémité extérieure de la première chambre 1A dans une narine). Ensuite, on enfonce le poussoir 7 sur toute la course H correspondant sensiblement à la hauteur de la bande de garantie.

Après un déplacement h, la pointe 6 a perforé la cloison 2 qui se déchire. L'air (ou un autre gaz) comprimé par le piston 5, éventuellement en plus d'une pression initiale, est libéré et traverse rapidement la première chambre 1A vers la sortie 3 en entraînant la poudre qui est projetée à l'extérieur. Le trou fait dans la cloison 2 provoque un effet Venturi, ce qui favorise l'entraînement de la poudre par l'air. L'appareil est jeté après usage. Sa structure extrêmement simple permet cette application dans le domaine médical.

On conçoit que la chambre 1A doit être plus petite que la chambre de gaz 1B pour avoir un bon balayage de la poudre. Cette première chambre 1A aura avantageusement une forme tubulaire sans rétrécissement pour favoriser l'entraînement de la poudre, pas nécessairement cylindrique. Le corps 1 présente près du poussoir 7 une collerette pour faciliter la prise de l'appareil et sa tenue pendant l'application. La pointe 6 peut présenter des rainures ou nervures longitudinales pour favoriser la déchirure de la cloison 2 et pour garantir le passage de l'air au cas où ladite cloison ne se déchire pas, mais est simplement perforée.

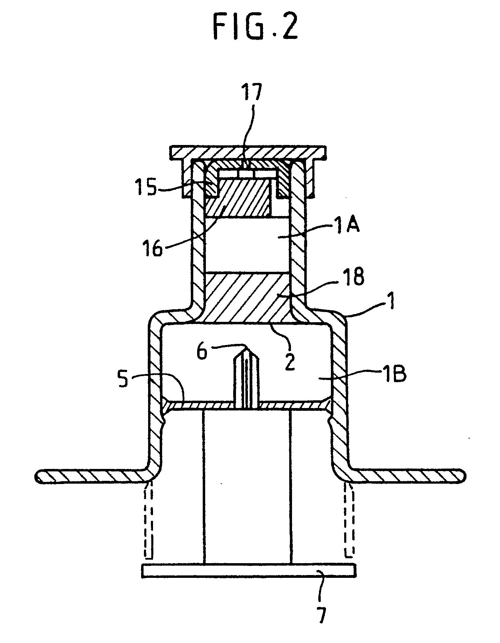

L'appareil de la figure 2 ne diffère de celui de la figure 1 qu'en ce qu'il comporte un gicleur de pulvérisation pour un liquide. Ce gicleur est dans cet exemple constitué d'une douille 15 qui peut être soudée ou collée à la paroi du corps 1, et un noyau est placé à l'intérieur pour coopérer avec le trou de sortie 17 de la douille 15. La chambre 1A est donc à l'origine remplie de liquide, avec éventuellement en plus un gaz.

Dans une variante d'application, il est possible d'utiliser une "bourre" 18, formant piston intermédiaire, poussée par l'air dès que la cloison 2 est percée par la pointe 6 du piston 5, la bourre poussant le liquide vers le gicleur. Ceci permet d'utiliser l'appareil dans n'importe quelle position.

Dans un mode d'utilisation de l'invention en rapport avec la variante de la figure 2, la seconde chambre 1B peut comprendre une partie de liquide et une partie de gaz. En tenant l'appareil avec la sortie vers le bas, et le piston en haut, lorsque la pointe 6 va percer l'opercule, l'air comprimé chassera le liquide de la chambre 1B dans la chambre 1A, les liquides se mélangeront, et le mélange, préparé ainsi au moment de l'emploi sera chassé par la pression de l'air à travers le gicleur. On peut donc ainsi projeter une dose constituée de deux produits dont le mélange n'est réalisé qu'au dernier moment, avant l'utilisation, et en fait, de façon quasi concommittante. En variante, la pointe 6 peut être proche de la cloison 2, et la seconde chambre 1B ne contient initialement pas de gaz sous pression. Ainsi, avec un faible déplacement du poussoir, on perce la cloison; on peut alors agiter l'appareil pour mélanger les deux liquides, et ensuite appuyer sur le poussoir pour projeter le mélange.

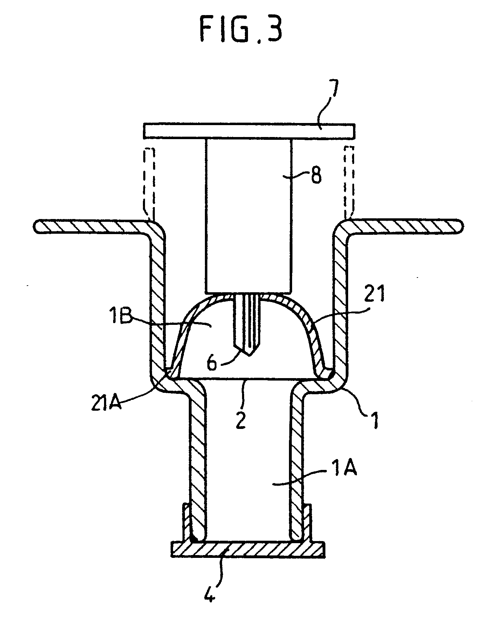

La figure 3 représente une variante dans laquelle la paroi déformable est constituée par une paroi sensiblement hémisphérique 21, en matériau souple, analogue à une poire. En appuyant sur le poussoir 7, l'extrémité large de la tige 8 vient appuyer sur la poire. L'écrasement de la poire provoque la montée de la pression, avant que la pointe 6 ne perfore la cloison 2.

Le bord périphérique 21A de la paroi souple 21 peut être collé ou soudé au corps du récipient 1. Cette paroi peut être moulée en une seule pièce avec la pointe 6. On obtient ainsi une étanchéité de haute qualité, et du gaz, air ou autre peut être renfermé pendant une longue période dans la chambre 1B, sans pertes sensibles. Le poussoir 7 et sa tige 8 peuvent aussi être moulés en une seule pièce avec la paroi 21 et la pointe 6, ou peuvent être surmontés sur cette paroi.

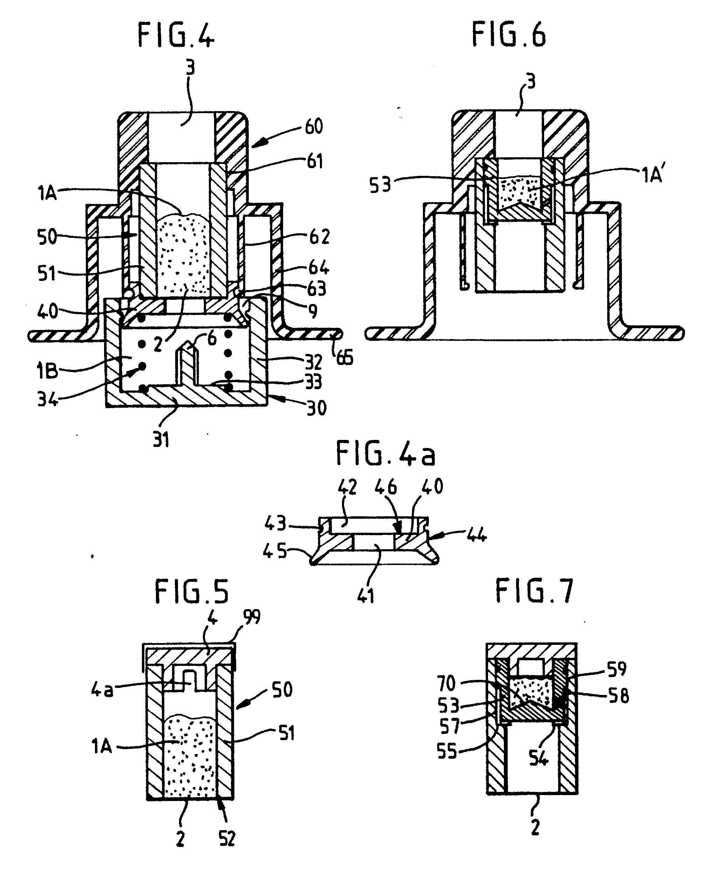

Les figures 4, 4a et 5 représentent une forme de réalisation de l'invention dans laquelle le dispositif de projection est rechargeable et plus particulièrement destiné à pulvériser des lyophilisats.

Ce dispositif se décompose en:
-un poussoir 30 comportant un fond cylindrique 31, lui-même comportant une face intérieure, une face extérieure et un bord périphérique extérieur, ledit poussoir 30 comportant aussi une paroi latérale cylindrique 32 s'étendant vers le haut à partir dudit bord périphérique extérieur, ladite paroi latérale cylindrique 32 comportant elle-même une surface intérieure sensiblement cylindrique, une surface extérieure cylindrique et une extrémité supérieure. Un épaulement cylindrique 33 est réalisé sur la face intérieure du fond 31, sensiblement centré sur cette face intérieure. Sur l'épaulement 33 est réalisé une pointe rainurée 6. La surface intérieure de la paroi latérale cylindrique 32 comporte en partie supérieure un bourrelet périphérique 9;
-un piston 40 de forme généralement cylindrique, adapté à coulisser verticalement à l'intérieur de ladite paroi latérale cylindrique 32, comportant une face supérieure, une face inférieure et une surface périphérique sensiblement cylindrique 44. Un trou cylindrique 41 sensiblement coaxial au piston 40 traverse ledit piston de haut en bas et est dimensionné pour laisser passer la pointe rainurée 6 lorsque le piston coulisse vers le bas. Un logement cylindrique 42 est réalisé dans la face supérieure du piston, sensiblement coaxial au trou cylindrique 41, s'étendant vers le bas à partir de ladite face supérieure jusqu'à une surface annulaire plane 46. Une rainure périphérique 43 est réalisée en partie supérieure de la surface périphérique cylindrique 44. Ladite surface périphérique 44 est prolongée vers le bas et vers l'extérieur par une lèvre d'étanchéité périphérique 45 s'appuyant contre la surface intérieure de la paroi latérale cylindrique 32. La lèvre d'étanchéité 45 est telle que le piston 40 puisse être inséré à l'intérieur du poussoir 30 en forçant la lèvre d'étanchéité 45 à passer au-dessus du bourrelet périphérique 9 lors du montage du dispositif, sans endommager ladite lèvre d'étanchéité 45;
-un ressort sensiblement hélicoïdal 34 placé à l'intérieur du poussoir 30, emboîté sur et retenu par l'épaulement cylindrique 33, et adapté à repousser le piston 40 vers le haut. La raideur du ressort 34 est telle qu'il puisse être comprimé à la force des doigts d'un utilisateur, et qu'il ne puisse pas forcer la lèvre d'étanchéité 45 du piston 40 à repasser par dessus le bourrelet périphérique 9 du poussoir 30;
-un flacon 50 de forme cylindrique comportant une paroi latérale cylindrique, en verre ou en plastique, elle-même composée d'une face supérieure, d'une face d'extrémité inférieure plane 52, d'une surface extérieure cylindrique et d'une surface intérieure cylindrique. Le flacon 50 comporte aussi une cloison déchirable 2 consistant avantageusement en un film thermoscellé sur la face d'extrémité inférieure plane 52 de la paroi latérale cylindrique 51. Ledit flacon 50 est inséré dans le logement cylindrique 42. L'intérieur dudit flacon 50 délimite la chambre 1A, la chambre 1B étant délimitée par le poussoir 30, le piston 40 associé au poussoir 30 par la lèvre d'étanchéité 45, et la cloison déchirable 2 venant en contact avec la face horizontale annulaire 46;

- un embout 60 de symétrie axiale, venant coiffer l'ensemble des composants décrits ci-dessus, comportant un orifice 3, un logement intérieur cylindrique 61 dans lequel vient se loger le flacon 50, au moins trois bras 62 s'étendant vers le bas et dotés à leur extrémité inférieure d'un ergot 63 adapté à s'encliqueter dans la rainure périphérique 43 du piston 40 sans que la force nécessaire à l'encliquetage ne comprime sensiblement le ressort 34, et tel que la force de traction nécessaire pour défaire l'encliquetage ne soit pas suffisante pour faire repasser la lèvre d'étanchéité 45 du piston par dessus le bourrelet périphérique 9, ledit embout 60 comprenant aussi un manchon périphérique cylindrique 64 s'étendant vers le bas, prolongé par une colerette 65 s'étendant radialement vers l'extérieur, destinée à favoriser la prise par un utilisateur.

Le piston 40, le flacon 50 et l'embout 60 sont configurés et dimensionnés de façon à ce que lorsque les bras 62 sont encliquetés dans la rainure périphérique 43, la face d'extrémité inférieure 52 de la paroi latérale cylindrique 51 et le film thermoscellé qui est solidaire de la face d'extrémité 52 soient appliqués contre la surface horizontale annulaire 46 du piston 40 de façon à réaliser une étanchéité. Ainsi, la chambre 1B est étanche.

Lorsqu'un utilisateur désire pulvériser une dose de produit, il prend un flacon 50 tel que représenté sur la figure 5, c'est-à-dire comportant un bouchon 4 et une capsule 99 déchirable pour pouvoir être stocké. L'utilisateur enlève alors la capsule 99 et le bouchon 4, place le flacon 50 dans le logement 42 du piston 40, puis coiffe l'ensemble à l'aide de l'embout 60 en encliquetant les ergots 63 des bras 62 dans la rainure périphérique 43 du piston 40. Le dispositif est alors prêt à être utilisé. La pulvérisation se fait en tenant l'embout 60 entre l'index et le majeur, et en appuyant sur le poussoir 30 à l'aide du pouce. La poussée comprime d'abord l'air compris dans la chambre 1B, puis la pointe rainurée 6 vient percer la cloison déchirable 2. L'air comprimé dans la chambre 1B s'engouffre alors dans la chambre 1A, et pulvérise le lyophilisat contenu dans la chambre 1A. Avant pulvérisation, le lyophilisat est sous une forme agglomérée ; c'est la violence de la projection par l'air comprimé qui le met sous forme pulvérulente. De ce fait, il est indispensable que le flacon 50 ne présente pas de rétrécissement et que l'orifice 3 soit au moins aussi large que l'intérieur du flacon 50, de façon à éviter un engorgement de l'orifice par le lyophilisat, et donc une mauvaise pulvérisation ou une pulvérisation impossible du produit.

Après pulvérisation, le ressort 34 remet le piston dans sa position initiale, l'embout 60 est démonté de l'assemblage par une simple traction, et le flacon 50 vide est jeté. Les autres composants de l'assemblage peuvent alors être réutilisés avec un nouveau flacon 50.

Lors de la fabrication du lyophilisat, une solution du produit à lyophiliser est placée dans le flacon 50 puis congelée à très basse température, puis réchauffée brutalement sous un vide très poussé, ce qui provoque la sublimation instantannée du solvant, généralement de l'eau. Durant ce processus, il est donc important que le flacon 50 et sa cloison déchirable 2 soient de bons conducteurs thermiques; de préférence, le flacon sera donc réalisé en verre, meilleur conducteur que le plastique, et la cloison déchirable sera réalisée en un complexe d'aluminium. Le complexe d'aluminium étant meilleur conducteur thermique que le verre, il est avantageux de donner à la cloison 2 une surface la plus grande possible.

Le fait de réaliser le flacon en verre et la cloison déchirable en complexe d'aluminium, favorise aussi la conservation du lyophilisat dans de bonnes conditions de sécheresse.

Le flacon 50 est doté d'un bouchon 4 comportant un couvercle à partir duquel s'étend une jupe cylindrique jusqu'à une extrémité inférieure. La jupe comporte une fente 4a sur une partie de sa hauteur, à partir de son extrémité inférieure. Pendant le processus de lyophilisation, le bouchon 4 est partiellement engagé, de façon à faire communiquer la chambre 1A avec l'extérieur par la fente 4a. A la fin de la lyophilisation, le bouchon 4 est engagé à fond pendant que le flacon est encore sous vide, ce qui ferme la chambre 1A.

La forme de réalisation représentée sur les figures 6 et 7 est destinée à la pulvérisation de poudres, c'est-à-dire de produits stockés sous forme pulvérulente.

Elle diffère de la précédente forme de réalisation par le fait que :

- la paroi latérale cylindrique 51 du flacon 50 comporte un logement cylindrique s'étendant à partir de la face d'extrémité supérieure vers le bas, jusqu'à une surface annulaire horizontale 55, ledit logement délimitant une surface intérieure cylindrique 57 ; et

- ledit logement cylindrique reçoit une coupelle cylindrique 53 comportant un fond, lui-même composé d'une surface périphérique cylindrique de dimension inférieure au diamètre de la surface intérieure cylindrique 57, d'une surface inférieure sensiblement plane et munie de nervures 54 en une périphérie extérieure, et une surface supérieure conique convexe 70. Les nervures 54 reposent sur la surface annulaire horizontale 55 de façon à laisser un passage entre ladite coupelle 53 et la surface annulaire 55. La coupelle 53 comporte aussi une paroi latérale cylindrique s'étendant à partir dudit fond vers le haut, et comportant une surface intérieure cylindrique, une surface extérieure cylindrique et une extrémité supérieure. La surface intérieure de la paroi latérale cylindrique et la surface supérieure du fond délimitent une chambre 1A′ contenant la poudre à pulvériser. La paroi latérale cylindrique de la coupelle 53 a un diamètre extérieur inférieur au diamètre de la surface intérieure cylindrique 57. Un ou plusieurs orifices non radiaux 58 traversent ladite paroi latérale cylindrique de la coupelle 53, en biais vers l'intérieur de la coupelle et vers le bas, débouchant à l'intérieur de ladite coupelle près de la base de la surface conique convexe 70. La paroi latérale cylindrique comporte aussi en partie supérieure de sa surface extérieure une lèvre d'étanchéité périphérique 59, dimensionnée et configurée pour être emboîtée en force à l'intérieur de la surface 57, de façon à ce que, lorsque de l'air comprimé pénètre dans le flacon 50 après percement de la cloison déchirable 2, la coupelle 53 ne soit pas éjectée vers l'extérieur. De préférence, le flacon 50 et la coupelle 53 sont de plus maintenus en place par l'embout 60.

Dans ces conditions, l'air comprimé passe alors entre les nervures 54, puis entre dans un espace annulaire compris entre la coupelle 53 et la surface intérieure 57, et pénètre dans la chambre 1A′ par l'intermédiaire du ou des orifices 58 dont la disposition par rapport à la surface conique convexe 70 entraîne un mouvement tourbillonnaire en même temps que l'expulsion de la poudre comprise dans la chambre 1A′.

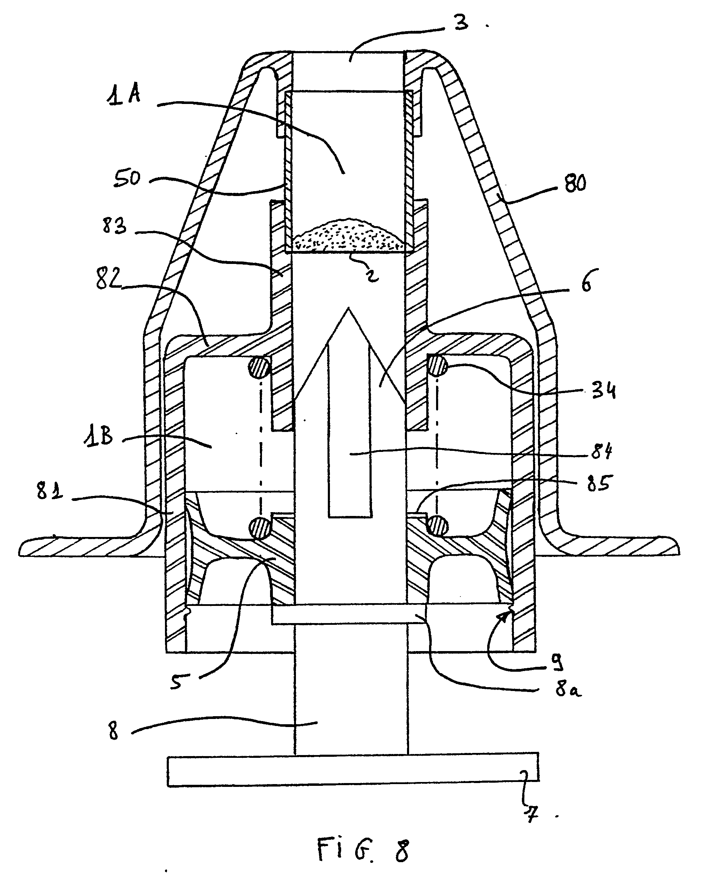

Une autre forme de réalisation est présentée à la figure 8. Dans cette forme de réalisation, la chambre 1B est formée par un cylindre 81 dans lequel coulisse un piston 5. Le piston 5 s'emboîte de façon étanche sur une tige 8 d'un poussoir 7, en butée contre un collet 8a de la tige. La tige 8 se prolonge au-delà du piston par une pointe 6 dotée de rainures 84. Le cylindre 81 comporte en une extrémité un fond 82 traversé par un tube 83 creux dans lequel la pointe 6 est adaptée à coulisser. Un ressort 34 s'appuie sur le fond 82 et sur le piston 5, de façon à éloigner le piston du fond. Un bourrelet périphérique intérieur 9 est formé en une extrémité inférieure du cylindre 81, de façon à empêcher le piston 5 de sortir du cylindre. Le tube 83 comporte en une extrémité supérieure un logement adapté à recevoir un flacon cylindrique 50 tel que décrit dans la forme de réalisation de la figure 5, en réalisant un emboîtement étanche. De plus, un embout 80 comportant l'orifice de sortie 3 vient s'emboîter sur le flacon 50. L'orifice 3 est de préférence de même largeur que la chambre 1A, délimitée par le flacon 50. L'embout 80 est prolongé radialement vers l'extérieur par une colerette facilitant la prise.

Lorsqu'un utilisateur appuie sur le poussoir 7, le piston 5 comprime l'air contenu dans la chambre 1B jusqu'à ce que la pointe 6 perce la cloison déchirable 2. L'air comprimé dans la chambre 1B s'échappe alors au travers des rainures 84 de la pointe 6 en pulvérisant le lyophilisat contenu dans la chambre 1A. En fin de course, le piston 5 vient en butée contre une extrémité du tube 83 par l'intermédiaire de nervures 85, qui laissent un passage libre entre la chambre 1A et la chambre 1B. Lorsque l'utilisateur relâche sa poussée, le piston 5 revient dans sa position initiale.


## Revendications

1.- Dispositif pour projeter et pulvériser une dose d'un produit divisé, en liquide ou en poudre, notamment à usage pharmaceutique, comportant un récipient (1) avec un orifice de sortie (3), une cloison déchirable (2) séparant le récipient en deux chambres, une première chambre (1A) du côté de l'orifice de sortie prévue pour recevoir le produit divisé, et une seconde chambre (1B) comportant au moment de l'emploi un gaz comprimé, ladite seconde chambre (1B) du récipient comportant une paroi (5) déformable en direction de la cloison déchirable par action d'un poussoir (7), la déformation de ladite paroi (5) ayant pour effet de réduire le volume de ladite seconde chambre (1B), afin d'y accroître la pression, caractérisé en ce que la paroi déformable (5) est munie d'une pointe rainurée (6) de façon que la déformation de la paroi amène la pointe à crever la cloison déchirable (2), ladite pointe étant disposée de façon à ne perforer la cloison (2) qu'après un déplacement h, de façon à faire monter la pression dans ladite chambre (1B) avant d'effectuer la perforation.

2.- Dispositif selon la revendication 1, caractérisé en ce que la paroi déformable est une paroi souple (21) de forme sensiblement hémisphérique, contre laquelle vient appuyer une extrémité d'une tige (8) du poussoir (7), et dont le bord est collé ou soudé au corps du récipient (1).

3.- Dispositif selon la revendication 2, caractérisé en ce que la paroi souple (21) est moulée en une seule pièce avec la pointe (6), éventuellement aussi avec le poussoir (7) et la tige (8).

4.- Dispositif selon la revendication 1, caractérisé en ce que la paroi déformable est un piston (5) contre lequel vient appuyer une extrémité d'une tige (8) du poussoir (7), la seconde chambre (1B) étant un cylindre adapté à ce piston.

5.- Dispositif selon la revendication 4, caractérisé en ce que ledit cylindre comporte un bourrelet périphérique intérieur (9) pour retenir le piston.

6.- Dispositif selon une des revendications 1 à 5, caractérisé en ce que le poussoir (7) est moulé avec une partie déchirable (11), dont la présence empêche son enfoncement.

7.- Dispositif selon une des revendications 1 à 5, caractérisé en ce que le récipient (1) est moulé avec une partie déchirable (11), dont la présence empêche l'enfoncement du poussoir.

8.- Dispositif selon une des revendications précédentes, caractérisé en ce que l'orifice de sortie (3) est muni d'un gicleur de pulvérisation (15,16) pour liquides, la première chambre (1A) contenant un premier liquide, et éventuellement en plus un gaz, la seconde chambre (1B) contenant un gaz, et éventuellement en plus un deuxième liquide, dont le mélange avec le premier ne se fait qu'au moment de l'utilisation.

9.-Dispositif selon une des revendications précédentes, caractérisé en ce que le récipient (1) est réalisé d'une seule pièce.

10.-Dispositif selon la revendication 1, caractérisé en ce que le récipient (1) se compose :
- d'un piston (40) comportant un trou axial (41) le traversant, une rainure périphérique (43) et une lèvre d'étanchéité périphérique (45);
- d'un embout (60) comportant l'orifice de sortie (3) et un moyen élastique (62, 63) pouvant s'encliqueter dans la rainure (43) du piston, l'embout et le piston définissant un logement pouvant recevoir un cartouche (50) comportant une paroi latérale (51) et la cloison déchirable (2), ledit flacon représentant l'enceinte de ladite première chambre (1A) contenant le produit à pulvériser, et une surface de contact entre le piston (40) et le cartouche (50) étant étanche;
et caractérisé en ce que la paroi déformable est un cylindre creux (30) doté d'un fond (31) et d'une paroi latérale cylindrique (32) à l'intérieur desquelles coulisse le piston (40) de façon à ce que la lèvre d'étanchéité (45) soit en contact avec ladite paroi latérale (32) sur toute une périphérie, la pointe rainurée (6) étant disposé sur le fond (31) du cylindre de façon à pouvoir passer au travers du trou (41) pour percer la cloison déchirable (2).

11.- Dispositif selon la revendication 10, caractérisé en ce qu'un ressort (34) sollicite le cylindre (30) de façon à l'éloigner du piston (40).

12.- Dispositif selon la revendication 11, caractérisé en ce qu'un bourrelet périphérique (9) est formé sur une surface intérieure du cylindre (30) de façon à ce qu'au montage du dispositif, le piston (40) puisse être inséré à l'intérieur du cylindre (30) en forçant la lèvre d'étanchéité (45) à passer sur le bourrelet (9), et en ce que la sollicitation du ressort (34) additionnée de la force de traction nécessaire à défaire l'encliquetage du moyen élastique (62, 63) de l'embout ne suffise à refaire passer la lèvre d'étanchéité (45) par dessus le bourrelet (9).

13.- Dispositif selon la revendication 1, caractérisé en ce qu'une première extrémité d'un cartouche (50) délimitant la chambre (1A) est emboîtée de façon étanche avec frottement à une extrémité de la chambre (1B), ladite première extrémité du cartouche (50) étant fermée par la cloison déchirable (2), et en ce qu'un embout (80) comportant l'orifice de sortie (3) est emboîté avec frottement sur une deuxième extrémité du cartouche (50).

14.- Dispositif selon la revendication 13 dans lequel la paroi déformable est un piston (5) contre lequel vient appuyer une extrémité d'une tige (8) du poussoir (7), la seconde chambre (1B) étant un cylindre adapté à ce piston, caractérisé en ce qu'un ressort (34) sollicite le piston (5) de façon à l'éloigner du cartouche (50).

15.- Dispositif selon une des revendications précédentes, caractérisé en ce que la cloison déchirable (2) est un film thermoscellé.

16.- Dispositif selon une des revendications précédentes, caractérisé en ce que la cloison déchirable (2) est réalisée dans un matériau conducteur de la chaleur.

17.- Dispositif selon la revendication 16, caractérisé en ce que la cloison déchirable (2) occupe toute une section de la chambre (1A), de façon à présenter une transmissivité thermique maximale.

18.- Dispositif selon une des revendications précédentes, caractérisé en ce que la chambre (1A) est remplie d'azote, en plus du produit à disperser.

19.- Dispositif selon une des revendications précédentes, caractérisé en ce que la chambre (1B) est remplie d'azote à une pression au moins égale à la pression atmosphérique.

20.- Dispositif selon une des revendications précédentes, caractérisé en ce que la chambre (1A) comporte une coupelle cylindrique (53) contenant une poudre à pulvériser et constituée d'un fond et d'une paroi latérale périphérique s'étendant verticalement à partir du fond, ladite paroi latérale périphérique comportant en partie supérieure un moyen d'étanchéité (59) emboîté en force de la chambre (1A), ladite paroi latérale périphérique comportant aussi au moins un orifice non radial (58) la traversant et dirigé en biais vers l'intérieur et vers le fond de la coupelle (53), ledit fond de la coupelle (53) comportant une surface supérieure conique convexe (70), la coupelle (53) coopérant avec la chambre (1A) de façon à permettre le passage de gaz comprimé de l'intérieur de la chambre (1A) vers l'intérieur de la coupelle (53) par l'intermédiaire du ou des orifice(s) (58) sans que la coupelle (53) ne soit éjectée vers l'extérieur, et le ou les orifice(s) (58) coopérant avec ladite surface conique (70) pour créer un mouvement tourbillonnaire en même temps que l'expulsion de la substance à pulvériser, lors du passage du gaz comprimé.

21.- Dispositif selon une des revendications 2 à 9, caractérisé en ce que le récipient (1) est en verre.

22.- Dispositif selon une des revendications 2 à 9, caractérisé en ce que le récipient (1) est en plastique.

23.- Dispositif selon une des revendications 10 à 14, caractérisé en ce que le cartouche (50) est en verre.

24.- Dispositif selon une des revendications 10 à 14, caractérisé en ce que le cartouche (50) est en plastique.

25.- Dispositif selon une des revendications précédentes, caractérisé en ce que la chambre (1A) présente une section constante.

26.- Dispositif selon la revendication 27, caractérisé en ce que l'orifice (3) de sortie est de même section que la chambre (1A).

27.- Dispositif selon une des revendications précédentes, muni d'un bouchon (4) comportant un couvercle à partir duquel s'étend une jupe jusqu'à une extrémité inférieure, ladite jupe s'emboîtant de façon étanche avec frottement dans la chambre (1A), caractérisé en ce que la jupe comporte une fente sur une partie de sa hauteur, à partir de son extrémité inférieure.

28.- Dispositif selon une des revendications précédentes, muni d'un bouchon pour fermer la chambre (1A), caractérisé en ce qu'une capsule métallique 99 déchirable est sertie par dessus le bouchon.

# FIG .1

# FIG.2

# FIG.3

# FIG.4

# FIG.6

# FIG.4a

# FIG.5

# FIG.7

FIG. 8